(19) Europäisches Patentamt
European Patent Office
Office européen des brevets

(11) EP 3 828 975 A1

(12) **EUROPÄISCHE PATENTANMELDUNG**

(43) Veröffentlichungstag:
**02.06.2021 Patentblatt 2021/22**

(51) Int Cl.:
***H01M 8/18*** *(2006.01)* ***C07C 46/06*** *(2006.01)*

(21) Anmeldenummer: **19212222.4**

(22) Anmeldetag: **28.11.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
**KH MA MD TN**

(71) Anmelder:
- **Technische Universität Graz**
  **8010 Graz (AT)**
- **Montanuniversität Leoben**
  **8700 Leoben (AT)**

(72) Erfinder:
- **SCHLEMMER, Werner**
  **8010 Graz (AT)**
- **SPIRK, Stefan**
  **8020 Graz (AT)**
- **KERN, Wolfgang**
  **8055 Seiersberg (AT)**
- **NOTHDURFT, Philipp**
  **4055 Pucking (AT)**
- **TAYLI, Melahat**
  **Mountain View, 94043**
  **California (US)**

(74) Vertreter: **Schwarz & Partner Patentanwälte OG**
**Patentanwälte**
**Wipplingerstraße 30**
**1010 Wien (AT)**

(54) **STABILE WÄSSRIGE ZUSAMMENSETZUNGEN UMFASSEND CHINONE UND DEREN VERWENDUNG IN REDOX-FLOW BATTERIEN**

(57) Die vorliegende Patentanmeldung betrifft eine wässrige Zusammensetzung umfassend
a) mindestens eine Verbindung mit der allgemeinen Formel

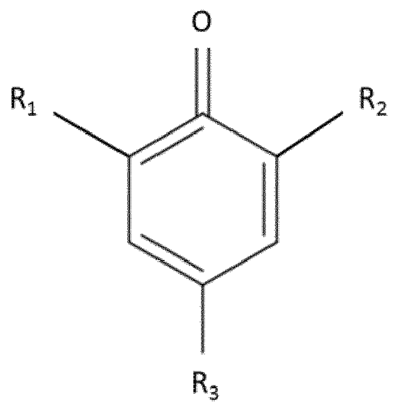

(I)

und mindestens eine Verbindung mit der allgemeinen Formel

EP 3 828 975 A1

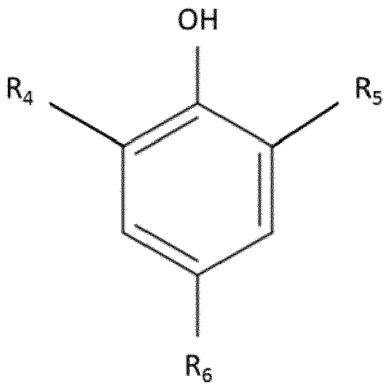

(II),

wobei $R_1$, $R_2$ und $R_3$ unabhängig voneinander O, H oder $OR_7$ und $R_4$, $R_5$ und $R_6$ unabhängig voneinander OH, H oder $OR_7$ sind, wobei $R_1$ O und $R_4$ OH, $R_2$ O und $R_5$ OH oder $R_3$ O und $R_6$ OH ist, wobei $R_7$ ein Alkyl- oder Allylrest ist, und b) Phosphationen und/oder Hydrogenphosphationen und/oder deren Kondensate.

**Beschreibung**

TECHNISCHES GEBIET

Die vorliegende Erfindung betrifft das Gebiet der Chinone und deren Verwendung in Redox-Flow-Batterien.

HINTERGRUND DER ERFINDUNG

In Redox-Flow-Batterien (RFBs) ist die volumetrische Kapazität gering, so dass derartige Batterien derzeit stationär und nicht mobil eingesetzt werden. Die Elektrolyte werden dabei in Tanks gelagert und durch eine Reaktionszelle gepumpt, wo diese oxidiert/reduziert werden. Somit ist es möglich, die Kapazität der Batterien durch Erhöhung des Volumens zu erhöhen (Leistungsdichte ca. 40 Wh/l). Großformatige Redox-Flow-Batterien (RFBs) können daher als Puffersysteme für das Stromnetz und als Backup-System für kritische Infrastrukturen verwendet werden. Einer der größten Nachteile der derzeit verwendeten Elektrolyte in RFBs ist deren Preis, die Nachhaltigkeit und deren Verfügbarkeit.

Hydrochinone sind Dihydroxyphenole, die in Wasser und mehreren organischen Lösungsmitteln löslich sind. Kürzlich rückten diese Verbindungen aufgrund ihres reversiblen elektrochemischen Verhaltens in den Fokus der Forschung und machten diese zu attraktiven Kandidaten für die Entwicklung von (wässrigen) RFBs. Mehrere Chinonderivate wie Sulfonate oder mit Alkylgruppen substituierte Chinone sowie ähnliche Verbindungen auf Basis von Naphtalen oder Anthracen wurden in den letzten Jahren beschrieben. Allerdings können insbesondere Chinone mit Gruppen mit +M-Effekten irreversible Nebenreaktionen eingehen, wodurch es zu Performance-Verlusten in RFB-Anwendungen kommt. Zudem ist hinreichend bekannt, dass diverse Chinone bzw. Chinonderivate insbesondere MQ, speziell im sauren Milieu instabil sind.

Es ist somit eine Aufgabe der vorliegenden Erfindung wässrige Zusammensetzungen umfassend Chinone bereitzustellen, die ausreichend stabil sind, um in Redox-Flow-Batterien eingesetzt zu werden.

ZUSAMMENFASSUNG DER ERFINDUNG

Die vorliegende Erfindung betrifft daher eine wässrige Zusammensetzung umfassend

a) mindestens eine Verbindung mit der allgemeinen Formel

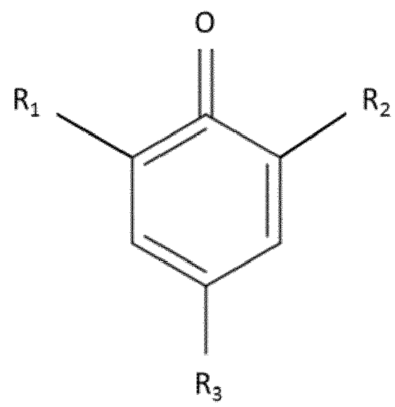

(I)

und mindestens eine Verbindung mit der allgemeinen Formel

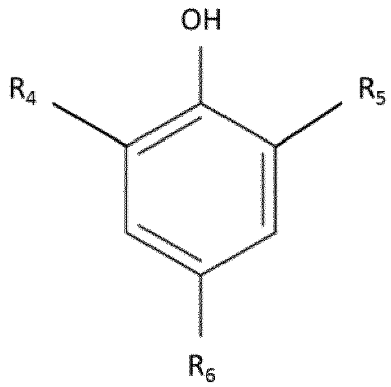

(II),

wobei $R_1$, $R_2$ und $R_3$ unabhängig voneinander O, H oder $OR_7$ und $R_4$, $R_5$ und $R_6$ unabhängig voneinander OH, H oder $OR_7$ sind, wobei $R_1$ O und $R_4$ OH, $R_2$ O und $R_5$ OH oder $R_3$ O und $R_6$ OH ist, wobei $R_7$ ein Alkyl- oder Allylrest ist, und

b) Phosphationen und/oder Hydrogenphosphationen und/oder deren Kondensate.

Es hat sich überraschender Weise gezeigt, dass die Anwesenheit von Phosphationen und/oder Hydrogenphosphationen bzw. deren Kondensate zu einer Stabilisierung von Verbindungen gemäß Formel I und Formel II in wässrigen Lösungen führt. Dies ist dahingehend von Vorteil, da es dadurch ermöglicht wird, wässrige Lösungen von Chinonen bereitzustellen, die ohne zusätzliche und aufwändige Maßnahmen stabil wären. Bisher wurde die Stabilität von Chinonen nur dadurch erreicht, indem man nicht-wässrige Lösungsmittel (wie z.B. MeCN) oder Inertgas (z.B. $N_2$ oder Argon) einsetzt. Da jedoch bei Anwendungen wie z.B. Redox-Flow-Batterien wässrige Lösungen zum Einsatz kommen, wären beide Maßnahmen praktisch nicht umsetzbar. Die in der erfindungsgemäßen wässrigen Zusammensetzung vorhandenen Verbindungen sind mehr als 30 Tage stabil, so dass die Zusammensetzung über einen längeren Zeitraum verwendbar ist.

Zudem hat sich erfindungsgemäß gezeigt, dass mit der erfindungsgemäßen Zusammensetzung eine Photoreduktion von Verbindungen gemäß Formel I zu Verbindungen gemäß Formel II möglich ist. Dies ist besonders überraschend, da bisher keine entsprechende Rückreaktion in einem wässrigen Medium, welches kein organisches Lösungsmittel wie z.B. Acetonitril umfasst, im Stand der Technik beschrieben ist. Dadurch hat die erfindungsgemäße Zusammensetzung auch und insbesondere für den Einsatz bei Redox-Flow-Batterien Vorteile.

Erfindungsgemäß ist einer der Substituenten $R_1$, $R_2$ und $R_3$ der Verbindung mit der Formel I O (Sauerstoff). Dementsprechend ist auch einer der Substituenten $R_4$, $R_5$ und $R_6$ der Verbindung mit der Formel II an der korrespondierenden Stelle OH. Daher ist $R_1$ O, wenn $R_4$ OH ist, $R_2$ ist O, wenn $R_5$ OH ist, und $R_3$ ist O, wenn $R_6$ OH ist.

Ein weiterer Aspekt der vorliegenden Erfindung betrifft die Verwendung einer erfindungsgemäßen wässrigen Zusammensetzung als Elektrolyt in einer Redox-Flow Zelle bzw. in einer Redox-Flow-Batterie.

Aufgrund der vorteilhaften Eigenschaften der erfindungsgemäßen Zusammensetzung eignet sich diese insbesondere für Redox-Flow Zellen. Vor allem die oben beschriebene Photoreduktion der in der wässrigen Lösung befindlichen Benzochinone ermöglicht die Herstellung von Redox-Flow Zellen bzw. Batterien, deren Halbzelle mittels Licht (z.B. Sonnenlicht, UV Strahlung usw.) geladen werden kann, um Verbindungen gemäß Formel (I) zu Verbindungen gemäß Formel (II) zu reduzieren. Zudem ist bei derartigen Anwendungen auch die Lagerstabilität der erfindungsgemäßen Zusammensetzung von Vorteil.

Ein noch weiterer Aspekt der vorliegenden Erfindung betrifft ein Verfahren zur Reduktion einer Verbindung mit der allgemeinen Formel

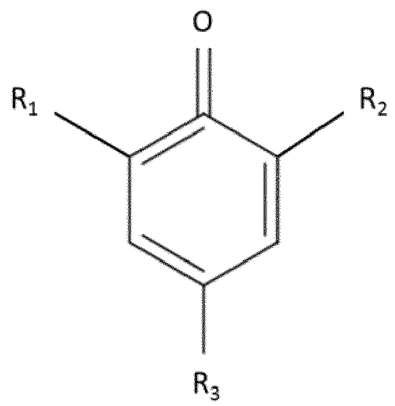

(I)

zu einer Verbindung mit der allgemeinen Formel

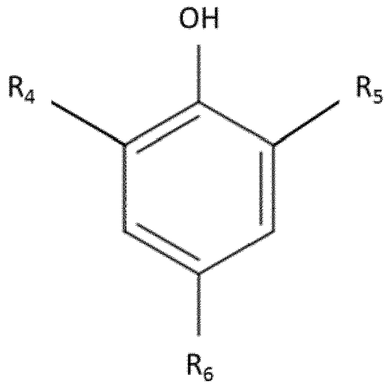

(II),

wobei $R_1$, $R_2$ und $R_3$ unabhängig voneinander O, H oder $OR_7$ und $R_4$, $R_5$ und $R_6$ unabhängig voneinander OH, H oder $OR_7$ sind, wobei $R_1$ O und $R_4$ OH, $R_2$ O und $R_5$ OH oder $R_3$ O und $R_6$ OH ist, wobei $R_7$ ein Alkyl- oder Allylrest ist, umfassend den Schritt des Inkontaktbringens einer erfindungsgemäßen wässrigen Zusammensetzung mit Licht.

Wie bereits oben angeführt, hat es sich überraschender Weise gezeigt, dass mit Hilfe der wässrigen Zusammensetzung gemäß der vorliegenden Erfindung Verbindungen mit der allgemeinen Formel (I) in Anwesenheit von Licht zu Verbindungen mit der allgemeinen Formel (II) reduziert werden können. Dies ermöglicht eine bei einer Oxidationsreaktion (z.B. in einer Redox-Flow-Batterie) aus einer Verbindung mit der allgemeinen Formel (II) hergestellten Verbindung mit der allgemeinen Formel (I) mit Lichtenergie zu regenerieren bzw. zu reduzieren.

KURZBESCHREIBUNG DER FIGUREN

Fig. 1 zeigt Nebenreaktionen in HCl: Durch Licht und Luftsauerstoff beschleunigte Oxidation von MHQ zu MQ und Bildung des MHQMQ Dimers in Abwesenheit von Phosphationen und/oder Hydrogenphosphationen und/oder deren Kondensate.

Fig. 2 zeigt ein 1H NMR des MQMHQ Dimers, welches in Anwesenheit von HCl gebildet wurde. 2-(2',5'-Dihydroxy-4'-Methoxyphenyl)-5-Methoxy-p-Benzochinon: 1 H NMR (300 MHz, DMSO-d6). δ: 3.74 (s, 3H, H-7); 3.80 (s, 3H, H-14); 6.15 (s, 1H, H-13); 6.48 (s, 1H, H-10); 6.59 (s, 1H, H-6); 6.76 (s, 1H, H-3); 8.46 (s, 1H, H-15), 9.24 (s, 1H, H-16).

Fig. 3a) zeigt eine Cyclovoltammetrie einer 10 mM MQ/MHQ Lösung in 1 M $H_3PO_4$ mit einer Scanrate von 25 mV/s um über 2 Stunden (100 Zyklen) um die Stabilität des wässrigen Gemisches zu zeigen. Fig. 3b) zeiget Cyclovoltammogramme derselben Lösung bei verschieden Vorschubgeschwindigkeiten (1000 mV/s, 800 mV/s, 500 mV/s, 300 mV/s, 150 mV/s, 100 mV/s, 50 mV/s with peak currents decreasing in this order) zur Bestimmung der Anzahl übertragener Elektronen. Fig. 3c) zeigt den Verlauf des Peakstroms Ip gegen die Wurzel der Scanrate einer 10 mM MQ/MHQ Lösung in 1M $H_3PO_4$ um die Linearität des Verlaufes als Grundlage der Berechnung für die Anzahl übertragener Elektronen zu zeigen. Fig. 3d) zeigt einen Linearen fit der Reaktionspotentialänderung E0 bei verschiedenen pH Werten. Die Steigung von 49 mV/pH deutet auf einen 2e⁻ Übergang hin.

Fig. 4 zeigt eine potentiostatische Cyclisierung einer Sandwich-Zelle mit Potentialen von 0,6 bzw. -0,6 V vs. Ag/AgCl. Die Potentiale wurden für jeweils 150 s angelegt. Als Aktivmaterialien wurden 200 μl einer 2 mM MHQ Lösung bzw. einer 4,5 mM MQ Lösung jeweils 0,5 M $H_3PO_4$ verwendet. Für bessere Klarheit wurden die Kurven normalisiert.

Fig. 5 zeigt ein galvanostatische Zyklisierung einer symmetrischen MQ/MHQ U-Zelle. Die Kapazität wurde auf die eingesetzte Masse des Aktivmaterials normalisiert. Dabei wurden 100 Zyklen bei einem Angelegten Strom von ±10 μA (0.5C) mit Umkehrpotentialen von 0.5/-0.5 V gemessen. Als Lösungen wurden 0.5 bzw. 1 mM MHQ bzw. MQ in 0.5M $H_3PO_4$ verwendet.

Fig. 6A zeigt die aromatische Region im 1H NMR Spektrum von MQ in 1M $D_3PO_4$ vor Bestrahlung mittels UV Lampe (Fig. 6A, unten) und die selbe Region nach Bestrahlung (64x 9 ns pulse bei 355 nm) mittels UV Lampe (siehe Fig. 6A oben). Die mit Rechtecken markierten Peaks zeigen die Bildung von MHQ. Fig. 6B zeigt ein cw-EPR Spektrum von MQ• in MeCN/wässrigem Puffer (pH = 3) 4:1, experimentell (oben) und simuliert (unten).

Fig. 7 zeigt schematisch den Aufbau einer Redox-Flow-Batterie (RFB) 1 umfassend zwei Tanks 2a und 2b, Pumpen 3a und 3b und eine Reaktionszelle 4 umfassend Elektroden 5a und 5b, Stromabnehmner 6a und 6b und eine Membrane 7.

Fig. 8 zeigt die Ergebnisse von Versuchen, die mit einer Redox-Flow-Batterie (RFB) durchgeführt wurden. Die unterbrochenen Linien zeigen die theoretische Kapazität an. Die Versuche wurden sowohl potentiostatisch (a) als auch galvoanostatisch (b) durchgeführt.

Fig. 9 zeigt den Strom- und Coulomb-Wirkungsgrad einer Redox-Flow Zelle mit 18,65 mg MQ und 25 mg pBQ als aktive Materialien in 35 ml 0,5 M $H_3PO_4$ bei potentiostatischen Zyklen im Bereich von ±0,75 V vs. CE mit 1600 s/Zyklus Lade-/Entladedauer.

BESCHREIBUNG DER AUSFÜHRUNGSFORMEN

"Wässrige Zusammensetzung", wie hierin definiert, bezieht sich auf Zusammensetzungen, die mehr als 50 Gew%, vorzugsweise mehr als 60 Gew%, noch mehr bevorzugt mehr als 70 Gew%, noch mehr bevorzugt mehr als 80 Gew%, noch mehr bevorzugt mehr als 90 Gew%, noch mehr bevorzugt mehr als 95 Gew%, Wasser umfassen. Vorzugsweise umfasst die erfindungsgemäße wässrige Zusammensetzung weniger als 20 Gew%, noch mehr bevorzugt weniger als 10 Gew%, noch mehr bevorzugt weniger als 5 Gew%, noch mehr bevorzugt weniger als 2 Gew%, noch mehr bevorzugt weniger als 1 Gew%, insbesondere kein nachweisbares, organisches Lösungsmittel, wie z.B. Acetonitril.

Die Substituenten $R_1$, $R_2$ und $R_3$ der Verbindungen mit der Formel I sind unabhängig voneinander O, H oder $OR_7$ und die Substituenten $R_4$, $R_5$ und $R_6$ der Verbindungen mit der Formel II sind unabhängig voneinander OH, H oder $OR_7$. In einer bevorzugten Ausführungsform der vorliegenden Erfindung sind die Substituenten $R_1$, $R_2$, $R_4$ und $R_5$ H oder $OR_7$.

Die Verbindungen der allgemeinen Formel I stellen vorzugsweise oxidierte Formen der Verbindungen mit der allgemeinen Formel II dar. Aus diesem Grund ist der Substituent $R_1$ vorzugsweise ident mit dem Substituenten $R_4$, Substituent $R_2$ vorzugsweise ident mit dem Substituenten $R_5$ und/oder Substituent $R_3$ vorzugsweise ident mit dem Substituenten $R_6$, sofern diese H und/oder $OR_7$ sind.

Gemäß einer bevorzugten Ausführungsform der vorliegenden Erfindung ist zumindest einer der Substituenten $R_1$, $R_2$ oder $R_3$ ein Sauerstoffatom (O), sodass die Verbindung mit der Formel I zumindest zwei Oxo-Gruppen aufweist. Dementsprechend weist die Verbindung mit der allgemeinen Formel II zwei Hydroxy-Gruppen an denselben Positionen auf.

Ist einer der Substituenten $R_1$, $R_2$ und $R_3$ ein O-Atom, ist dieser Substituent mittels einer Doppelbindung am entsprechenden C-Atom des Ringes gebunden.

In einer weiteren bevorzugten Ausführungsform ist $R_7$ sowohl in der Verbindung mit der Formel I als auch in der Verbindung mit der Formel II ident.

Substituent $R_7$ ist ein Alkylrest oder ein Allylrest. Der Alkylrest ist vorzugsweise ein $C_1$ bis $C_5$ Alkylrest. Der Allylrest ist vorzugsweise ein $C_2$ bis $C_5$ Allylrest. "$C_1$ bis $C_5$ Alkylrest" und "$C_2$ bis $C_5$ Allylrest", wie hierein verwendet, umfasst Alkyl- bzw. Allylreste mit 1 bzw. 2 bis 5 Kohlenstoffresten, die vorzugsweise linear angeordnet sind (d.h. ein linearer Alkyl- bzw. Allylrest). Gemäß einer bevorzugten Ausführungsform der vorliegenden Erfindung ist Substituent $R_3$ ein Methylrest, ein Ethylrest, ein Vinylrest, ein Propylrest, ein Propylenrest, ein Butylrest, eine Butenylrest, ein Pentylrest oder ein Pentenylrest, wobei Substituent $R_7$ besonders bevorzugt ein Methylrest ist.

Die erfindungsgemäße wässrige Zusammensetzung umfasst Phosphationen und/oder Hydrogenphosphationen. Diese werden vorzugsweise in Form von Phosphorsäure oder Salzen der Zusammensetzung zugesetzt. "Kondensate" von Phosphationen und/oder Hydrogenphosphationen können durch Wasserabspaltung gebildet werden. Dabei entstehen Diphosphationen bzw. poly- oder cyclo-Phosphationen.

Gemäß einer bevorzugten Ausführungsform der vorliegenden Erfindung ist die Verbindung der Formel (I) ein Benzochinon und die Verbindung der Formel (II) ein Hydrochinon.

Gemäß einer besonders bevorzugten Ausführungsform der vorliegenden Erfindung ist die Verbindung der Formel (I) 2-Methoxybenzochinon (MQ) oder Parabenzochinon (pQ) und die Verbindung der Formel (II) 2-Methoxyhydrochinon (MHQ) oder Parahydrochinon (pHQ).

Es hat sich erfindungsgemäß gezeigt, dass Phosphationen und/oder Hydrogenphosphationen bzw. deren Kondensate insbesondere wässrige Zusammensetzungen umfassend MQ, pHQ, MHQ und/oder pQ stabilisieren. Die wässrige Zusammensetzung der vorliegenden Erfindung umfasst vorzugsweise MQ und MHQ und/oder pQ und pHQ.

Gemäß einer weiteren bevorzugten Ausführungsform der vorliegenden Erfindung sind die Kondensate der Phosphationen und Hydrogenphosphationen Hydrogendiphosphationen, Hydrogentriphosphationen, Hydrogenoligophosphationen oder Hydrogenpolyphosphationen.

Gemäß einer bevorzugten Ausführungsform der vorliegenden Erfindung umfasst die wässrige Zusammensetzung 0,0001 bis 1,2 mol/L, vorzugsweise 0,001 bis 1 mol/L, noch mehr bevorzugt 0,05 bis 0,75 mol/L, noch mehr bevorzugt 0,1 bis 0,75 mol/L, noch mehr bevorzugt 0,2 bis 0,7 mol/L, einer Verbindung der Formel I und/oder II. Diese Konzentrationsangaben sind vorzugsweise die Summe der Verbindungen mit den Formeln I und II in der erfindungsgemäßen Zusammensetzung. Das Verhältnis der Verbindungen mit den Formeln I und II beträgt vorzugsweise 1:10 bis 10:1, noch mehr bevorzugt 1:5 bis 5:1, noch mehr bevorzugt 1:3 bis 3:1, noch mehr bevorzugt 1:2 bis 2:1, besonders bevorzugt ca. 1:1.

Gemäß einer besonders bevorzugten Ausführungsform der vorliegenden Erfindung umfasst die Zusammensetzung in Summe 0,01 bis 0,75 mol/L, vorzugsweise 0,05 bis 0,7 mol/L, noch mehr bevorzugt 0,1 bis 0,6 mol/L, noch mehr bevorzugt 0,15 bis 0,5 mol/L, Phosphationen und/oder Hydrogenphosphationen und/oder deren Kondensate.

Gemäß einer bevorzugten Ausführungsform der vorliegenden Erfindung umfasst die Zusammensetzung zusätzlich mindestens eine Säure mit einem pKs-Wert von -7,5 bis 8, vorzugsweise -3,5 bis 8, noch mehr bevorzugt 1,5 bis 8, noch mehr bevorzugt von 2 bis 7,5, noch mehr bevorzugt von 4,2 bis 6,8, noch mehr bevorzugt von 4,5 bis 6.

Um den pH-Wert der erfindungsgemäßen Zusammensetzung zu kontrollieren bzw. zu reduzieren, umfasst die Zusammensetzung vorzugsweise eine Säure mit einem pKs-Wert von weniger als 8.

Gemäß einer weiteren bevorzugten Ausführungsform der vorliegenden Erfindung ist die Säure eine organische oder anorganische Säure, wobei die anorganische Säure vorzugsweise ausgewählt ist aus der Gruppe bestehend aus Salzsäure, Schwefelsäure, Salpetersäure und Phosphorsäure, und die organische Säure vorzugsweise ausgewählt ist aus der Gruppe bestehend aus Ameisensäure, Zitronensäure, Essigsäure, Propionsäure, Buttersäure, Glyoxalsäure, Malonsäure, Bernsteinsäure, Glutarsäure, Adipinsäure, Zitronensäure, Trimesinsäure, Trimellitsäure und Halbester davon.

Gemäß einer bevorzugten Ausführungsform der vorliegenden Erfindung umfasst die Zusammensetzung 0,001 bis 5 mol/L, vorzugsweise 0,005 bis 3 mol/L, Säure.

Gemäß einer weiteren bevorzugten Ausführungsform der vorliegenden Erfindung weist die Zusammensetzung einen pH-Wert von weniger als 7, vorzugsweise von 1 bis 6,9, auf.

Gemäß einer besonders bevorzugten Ausführungsform der vorliegenden Erfindung umfasst die Zusammensetzung mindestens einen Elektrolyten ausgewählt aus der Gruppe bestehend aus der Gruppe der Alkali- und Erdalkalimetallchloride, vorzugsweise NaCl, KCl oder $CaCl_2$, aus der Gruppe der Alkali- und Erdalkalimetallsulfate, vorzugsweise $Na_2SO_4$ oder $K_2SO_4$, und der Gruppe der Alkali- und Erdalkalimetallsalze von Carbonsäuren, Dicarbonsäuren und Tricarbonsäuren, vorzugsweise Natriumacetat, Natriumcitrat, Calciumcitrat oder Natriumoxalat.

Die erfindungsgemäße Zusammensetzung kann als Elektrolytlösung in Redox-Flow Zellen bzw. Batterien eingesetzt werden. Daher umfasst die erfindungsgemäße Zusammensetzung mindestens einen Elektrolyten.

Gemäß einer bevorzugten Ausführungsform der vorliegenden Erfindung umfasst die Zusammensetzung mindestens einen Elektrolyten in einem Konzentrationsbereich von 0,001 bis 1,5 mol/L.

Ein weiterer Aspekt der vorliegenden Erfindung betrifft die Verwendung einer erfindungsgemäßen wässrigen Zusammensetzung als Elektrolyt in einer Redox-Flow Zelle.

Ein noch weiterer Aspekt der vorliegenden Erfindung betrifft eine Redox-Flow Zelle umfassend eine erfindungsgemäße wässrige Zusammensetzung.

Redox-Flow Zellen, auch als Redox-Flow Batterien oder als Redox-Fluss Batterien bezeichnet, können zur Speicherung von elektrischer Energie eingesetzt werden. Redox-Flow Zellen enthalten zwei polaritätsspezifische Kammern, in denen jeweils eine redoxaktive chemische Verbindung bzw. eine redoxaktive Verbindung in beiden Kammern in gelöster Form vorliegt und mit einem Flüssigkeitsspeicher in Verbindung steht. Auf diese Weise werden zwei unabhängige Kreisläufe für die beispielsweise in Wasser oder organischem Lösungsmittel gelösten redoxaktiven Verbindungen gebildet, welche durch eine Membran zwischen den polaritätsspezifischen Kammern getrennt sind. Über diese Membran erfolgt ein Ionenaustausch zwischen den beiden Kammern. Die Zellen eignen sich insbesondere für stationäre Speicheranwendungen, zum Beispiel als Pufferbatterie für Windkraftanlagen beziehungsweise als Leistungs- und Regelreserven zum Lastausgleich in Stromnetzen, können aber durchaus auch als mobile Energiespeicher, zum Beispiel für den Betrieb von Elektroautos und elektronischen Geräten, eingesetzt werden.

Die zur Potentialeinstellung an den Elektroden benötigten Verbindungen sind gelöste, redoxaktive Verbindungen, die beim Lade- bzw. Entladevorgang in einem elektrochemischen Reaktor in ihre jeweils andere Redoxstufe überführt werden. Dazu werden die Elektrolytlösungen (Katholyt, Anolyt) aus einem Tank entnommen und aktiv an die Elektroden gepumpt. Anoden- und Kathodenraum sind im Reaktor durch eine ionenselektive Membran getrennt, welche meist eine hohe Selektivität für Protonen zeigt. Solange Elektrolytlösung gepumpt wird, kann Strom entnommen werden. Während des Ladevorgangs einer Redox-Flow Zelle wird üblicherweise der Pumpvorgang umgekehrt. Damit ist die Energiemenge, die in einer Redox-Flow Zelle gespeichert werden kann, direkt proportional zur Größe der Vorratstanks. Die entnehmbare Leistung dagegen ist eine Funktion der Größe des elektrochemischen Reaktors.

Die erfindungsgemäße wässrige Zusammensetzung kann als Elektrolyt in Redox-Flow Zellen eingesetzt werden. Diese Zusammensetzung ist insbesondere vorteilhaft, da zur Gänze auf organische Lösungsmittel verzichtet werden kann. Zudem hat sich gezeigt, dass die Regenerationsreaktion mittel Licht durchgeführt werden kann. Dadurch wird es ermöglicht den Elektrolyten einer Redox-Flow Zelle mittels Lichtenergie zu regenerieren. Dementsprechend ist es von Vorteil den Elektrolytspeicher umfassend die erfindungsgemäße wässrige Zusammensetzung zumindest lichtdurchlässig, vorzugsweise UV-durchlässig, zu gestalten, um die Regeneration, d.h. Reduktion, der Verbindungen mit der allgemeinen Formel I zu Verbindungen mit der allgemeinen Formel II zu ermöglichen.

Die erfindungsgemäße Zusammensetzung wird vorzugsweise als Katholyt in Redox-Flow Zellen verwendet. Als Anolyt können wässrige Zusammensetzungen umfassend Verbindungen wie sulfonierte Chinone (E0=-200 mV vs Ag/AgCl), NADH (E0=-500 mV vs. Ag/AgCl) oder Schwermetallsalze mit einem starker negativen Redoxpotential (z.B. $V^{3+}/V^{2+}$ mit E0=-470 mV vs. Ag/AgC1) verwendet werden. Die erfindungsmäßige Zusammensetzung kann bei entsprechender Gegenseite ebenfalls als Anolyt herangezogen werden. Zusätzlich eignen sich auch wässrige Zusammensetzungen umfassend Anthrachinone (sulfoniert, alkyliert und/oder acyliert), Naphtachinone (sulfoniert, alkyliert und/oder acyliert), Viologene, 2,2,6,6-Tetramethylpiperidinyloxyl (TEMPO) und Derivate davon, redoxaktive Polymere, insbesondere Polyanillin mit Viologen-, Pyridinyl- und/oder Chinonstrukturen, natürliche Chinone, insbesondere Tannine, Tanninsäure, Gallsäure, Kaffeesäure und/oder Chlorogensäure, Porphyrine und/oder Pyrrole als Anolyte.

Ein weiterer Aspekt der vorliegenden Erfindung betrifft ein Verfahren zur Reduktion einer Verbindung mit der allgemeinen Formel

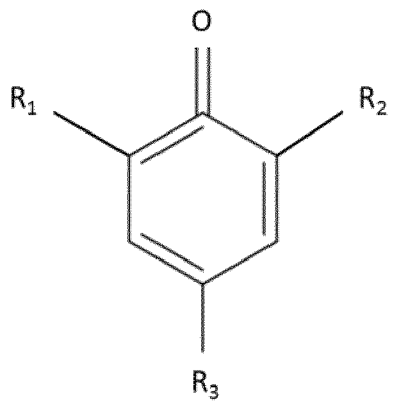

(I)

zu einer Verbindung mit der allgemeinen Formel

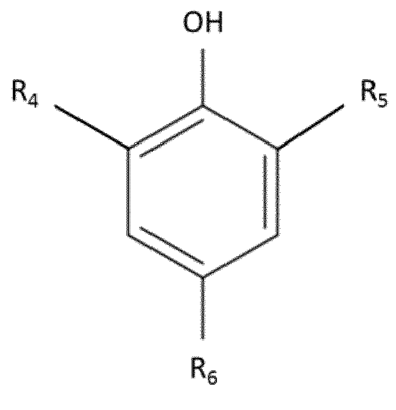

(II),

wobei $R_1$, $R_2$ und $R_3$ unabhängig voneinander O, H oder $OR_7$ und $R_4$, $R_5$ und $R_6$ unabhängig voneinander OH, H oder $OR_7$ sind, wobei $R_1$ O und $R_4$ OH, $R_2$ O und $R_5$ OH oder $R_3$ O und $R_6$ OH ist, wobei $R_7$ ein Alkyl- oder Allylrest ist, umfassend den Schritt des Inkontaktbringens einer erfindungsgemäßen wässrigen Zusammensetzung.

Das erfindungsgemäße Verfahren, d.h. die Reduktion einer Verbindung mit der allgemeinen Formel I zu einer Verbindung mit der allgemeinen Formel II, kann in Anwesenheit von Licht in der erfindungsgemäßen Zusammensetzung durchgeführt werden. Dadurch ist es möglich eine Redox-Flow Zelle durch bloße Lichteinstrahlung (z.B. durch Sonnenlicht/Licht im sichtbaren Bereich und/oder UV Strahlung) aufzuladen.

Das eingebrachte Licht weist vorzugsweise eine Wellenlänge von 190 bis 800 nm, vorzugsweise von 190 bis 400 nm, noch mehr bevorzugt von 350 bis 400 nm, auf.

Gemäß einer bevorzugten Ausführungsform der vorliegenden Erfindung ist das Licht Sonnenlicht und/oder UV Licht ist.

Es hat sich gezeigt, dass die Reduktion in einem Temperaturbereich von 5 bis 65°C, vorzugsweise von 15 bis 55°C, noch mehr bevorzugt von 25 bis 35°C, besonders vorteilhaft ist.

Da die Erfindungsgemäße Zusammensetzung zur Verwendung bei Redox-Flow Zellen besonders gut geeignet ist, wird die Reduktion vorzugsweise in einer Redox-Flow Zelle durchgeführt, insbesondere jedoch nicht im Elektrodenbereich sondern beispielsweise in einem Vorratstank, in der die Zusammensetzung gelagert wird, so dass die Regenerationsreaktion, d.h. die Reduktion, nicht die Stromerzeugung beeinflusst.

Die vorliegende Erfindung wird anhand der folgenden Beispiele eingehender erläutert, ohne jedoch auf diese beschränkt zu sein.

BEISPIELE

In den folgenden Beispielen wird gezeigt, dass ein Chinon-System umfassend 2-Methoxyhydrochinon (MHQ) und 2-Methoxybenzochinon (MQ) über 1 Monat in einem Lösungsmittelsystem mit Phosphorsäure, vorzugsweise 0,3-0,8 M mit Phosphorsäure, stabil ist. Bei der elektrochemischen Analyse konnte eine reversible Zwei-Elektronen-Oxidation/Reduktion nachgewiesen werden, die Redox-Potenziale von ca. 380 mV vs. Ag/AgCl aufweist. Dies allein würde das MQ/MHQ-System als Posolyt (Katholyt) für großtechnische RFB-Anlagen attraktiv machen.

Es konnte zudem nachgewiesen, dass das oben beschriebene System in Phosphorsäure mit Licht aufgeladen werden kann (MQ $\rightarrow$ MHQ). Bereits kurze UV-Lichtimpulse von 30 Sekunden reichen aus, um eine signifikante Menge an MQ zu reduzieren. Laborversuche zeigen zudem, dass auch Sonneneinstrahlung zu einer partiellen Reduktion von MQ führt. Daher wurden mehrere (radikale) Abbaumechanismen in $H_3PO_4$, HCl und $H_2SO_4$ mit Hilfe von CIDNP-NMR, 1H NMR, cw-EPR-Spektroskopie sowie Kristallographie untersucht. Der günstige Beschaffungspreis und die Verfügbarkeit machen MHQ und MQ zu geeigneten Kandidaten für aktive Verbindungen in großen Energiespeichern.

***Beispiel 1:* *Stabilität von 2-Methoxyhydrochinon (MHQ) und 2-Methoxybenzochinon (MQ)***

Es ist in der Fachwelt hinreichend bekannt, dass Lösungen mit MQ und MHQ relativ labil sind. Während MQ und MHQ einzeln in saurer, wässriger Lösung kaum Nebenreaktionen zeigen, neigen Gemische der beiden stark zu Degradationen. Die am häufigsten vorkommenden Nebenreaktionen sind Dimerisierungen. Dabei agiert MHQ als Nukleophil und MQ als Elektrophil. Besonders problematisch ist, dass MHQ in sauren Medien zur Oxidation zu MQ neigt, was die Lagerstabilität stark beeinflusst. Einen starken Einfluss auf die Stabilität hat zudem Licht: MQ kann durch Absorption in einen (reaktiven) angeregten Zustand überführt werden. Dadurch sind auch radikalische Nebenreaktionen nicht auszuschließen.

Um die Stabilität von MQ und MHQ Gemischen zu untersuchen, wurden mehrere Säuren und Stabilisatoren getestet, wobei Gemische mit Phosphorsäure am stabilsten waren. In Fig. 1 sind Nebenprodukte in HCl und $H_3PO_4$ aufgezeigt, welche mittels 1H NMR bzw. XRD charakterisiert wurden. Für die NMR Messungen wurden die entsprechenden mittels eines Bruker NMR mit 300 MHz bei 4 s delay vermessen (8 Scans/Probe). Dafür wurden 5 mg der Proben in 0.8 mL DMSO-d6, $CDCl_3$ oder $D_2O$ gelöst. Die Spektren wurden auf die Lösungsmittelsignale bei 2.50 ppm für DMSO, 7.23 für $CDCl_3$ sowie 4.80 ppm für $D_2O$ referenziert. Die Daten wurden mittels der TopSpin software (Varian) analysiert.

Während oxidierende Säuren wie HCl zu MHMQH (Fig. 2) und eventuell weiter zu MQMQ Dimeren führten, war die einzige Nebenreaktion, die in Anwesenheit von Phosphorsäure beobachtet wurde, die Bildung des MHQMHQ Dimers in Form von schwarzen Kristallen. Diese Reaktion konnte durch geringe Konzentrationen von MQ und MHQ (=< 0,5 mM), Lichtausschluss, Luftausschluss und langsame Zugabe der Chinone zur sauren Lösung minimiert werden. Der Grund für die gute Stabilität in $H_3PO_4$ könnte die Fähigkeit von o-Phosphorsäure sein, Radikale durch die Bildung von Phosphatoligomeren abzufangen sowie die antioxidative Wirkung dieser Säure.

***Beispiel** 2: Elektrochemie von 2-Methoxyhydrochinon*

Unter sauren Bedingungen konnte 2-Methoxyhydrochinon zu 2-Methoxybenzochinon oxidiert und dann wieder zu MHQ reduziert werden. Dabei handelt es sich um einen 2 $e^-$ Übergang gemäß einem CCEE Mechanismus (siehe Fig. 3). Für die Bestimmungen wurde ein 3-Elektrodenaufbau, bestehend aus einer Arbeitselektrode (Glas-Kohlenstoff), einer Gegenelektrode (Pt Draht) sowie einer Referenzelektrode (Ag/AgCl, $E_0$=195 mV vs. SHE) verwendet. Die Scanrate war, sofern nicht anders angegeben, 100 mV/s in einem Bereich von typischer Weise -0,75 -0.75 V. Die Experimente wurden mit Hilfe eines Gamry PCI4-300 controller boards durchgeführtund mit der Gamry Elchem Analyst Software analysiert. Auf die Zugabe von Leitsalzen wurde gänzlich verzichtet. Abhängig vom pH-Wert der Lösung lag das Redox-Potential zwischen 328 mV (pH 0.5) und 307 mV (pH 1.5) gegen eine Ag/AgCl Referenzelektrode.

Messungen über eine längere Zeit (siehe Fig. 3a) zeigten einen stabilen, reversiblen Verlauf der Oxidation und Reduktion. Sowohl die Aufnahme von Cyclovoltammogrammen (via Diffusionslimitierung; siehe A.J. Bard, "Electrochemical methods: fundamentals and applications"; Allen J. Bard, Larry R. Faulkner, Wiley, New York, 1980) als auch die Messungen bei verschiedenen pH-Werten ("shift" des Redoxpotentials E0; siehe Weber AZ et al. J. Appl. Electrochem. 41(2011):1137) deuten auf den angenommenen $2e^-$ Übergang hin. Die Messung wurde im oben angegebenen Aufbau mit 25 mV/S Scanrate zwischen -0,25 und 0,75 V vs. Ag/AgCl durchgeführt.

Die Gleichungen 1 und 2 zeigen die Berechnung der Anzahl übertragener Elektronen mittels CV (Cyclovoltammetrie). Darin enthalten sind der Diffusionskoeffizient D [$cm^2s^{-1}$], die Elektrodenoberfläche A [$cm^2$], die Faraday Konstante F [C/mol], die Vorschubrate v [$Vs^{-1}$] der Peakstrom iP [mA], die Anzahl übertragener Elektronen n [], die Konzentration c [mol ml-1], die ideale Gaskonstante R [$Jmol^{-1}$ $K^{-1}$] und die Temperatur T [K]. Die entsprechenden Messungen wurden im oben beschriebenen 3-Elektrodenaufbau durchgeführt; die Scanraten wurden waren 50, 100, 150, 300, 500 und 1000 mV/s in Potentialbereichen zwischen -1 und 1 V.

$$D = \left(\frac{i_P}{2.7*10^5*A*c}\right)^2 * \frac{1}{\nu}$$

Gleichung (1)

$$i_p = 0.4463 * n * F * A * c * \left(\frac{n*F*\nu*D}{R*T}\right)$$

Gleichung (2)

Messungen bei verschiedenen pH-Werten unterstützen die $2e^-$ Hypothese, da der Shift des Redoxpotentials bei Chinonen meist um -22 mV/pH für 1 e- und -59 mV/pH für 2 e-Übergänge liegt (siehe A.J. Bard, "Electrochemical methods: fundamentals and applications"; Allen J. Bard, Larry R. Faulkner, Wiley, New York, 1980).

***Beispiel** 3: Tests von MQ/MHQ basierten Vollzellen*

MQ/MHQ wies wie unter Beispiel 2 gezeigt bei pH 0,5 bis 1 ein Redoxpotential von ca. 0,33 V vs. Ag/AgCl auf. Bei Nutzung eines kommerziell erhältlichen Chinons (z.B. einem monosulfonierten p-Benzochinon) mit einem Redoxpotential von ca. 0 V vs. Ag/AgCl oder NADH mit einem Potential von ca. -0,5 vs. Ag/AgCl bei pH 1 auf der Gegenseite einer Redox-Flow-Zelle wäre so eine Spannungsdifferenz von 0,35 V bzw. 0,8 V in der Zelle zu erreichen. Als alternative Substanzen können z.B. Eisensalze wie z.B. $V^{3+}/V^{2+}$ mit einem Redoxpotential von ca. -480 mV gegen Ag/AgCl angewandt werden, was eine Nettospannung von ca. 0,8 V ergeben würde. Die resultierende volumetrische Energiedichte hängt zusätzlich von der Löslichkeit der Chinone im betreffenden Medium ab. Bei einer (für Chinone klassischen) Löslichkeit von ca. 0,7 mol/l in saurer Lösung würde sich eine Energiedichte von etwa 30 Wh/l ergeben.

Um die elektrochemische Performance von MQ und MHQ zu testen, wurden symmetrische Vollzellen mit Referenz-MHQ und daraus synthetisiertem MQ als Gegenseite (im Überschuss) hergestellt. Dies minimiert auch Probleme durch Cross-over Phänomene. Dafür wurden Sandwich Zellen konstruiert, um die Elektrochemie im kleinen Maßstab zu analysieren, U-Typ Zellen für das Verhalten in statischen Systemen sowie volle Flusszellen (siehe z.B. Fig. 7). Für diese Experimente wurden 10 mg von MQ und 10 mg MHQ jeweils in 1 mL 0,7; 0,75 bzw. 1M $H_3PO_4$ gelöst. Die jeweilige MQ bzw. MHW Lösung wurde auf Kohlenstoffpapierelektroden aufgetropft die in ein Gehäuse aus gepresstem Graphit eingespannt wurden. Die 2 Elektroden wurden durch eine Nafion 211 Membran getrennt und die Spannung an die Graphitgehäuse angelegt. Die Messungen erfolgten im Potentiostatischen Modus ($\pm$0,75 V vs. Gegenelektrode, 3 min/Ladung/Entladung).

Die Auswertung der Sandwich-Zelle in 0,5 M $H_3PO_4$ zeigt einen relativ konstanten Verlauf über 50 Zyklen bei

einer Coulomb-Effizienz von über 95% für alle Zyklen (siehe Fig. 3). Bei weiteren Zyklen erfolgt in dieser Anordnung ein trockenlaufen der Membran was zu höheren Verlusten führt. Auf Grund von Elektrolytverlusten können die Absolutwerte der Kapazitäten abweichen, darum wurden sie jeweils auf den Maximalwert normalisiert. Die theoretische Kapazität der Zellen liegt bei ca. 1,4 Ah/g Aktivmaterial.

Ein ähnliches Ladungs-/Entladungsschema wie in der Sandwichzelle kann in gläsernen Zellen ohne Fluss und Rührung beobachtet werden mit (nach den ersten Zyklen) geringen Verlusten (siehe Fig. 4). Dafür wurden 10 mg MQ bzw. 10 mg MHQ in jew. 3 mL 0,5M $H_3PO_4$ gelöst. Zwei Glasrohre (3 mm Durchmesser, 6 cm Länge) wurden in der Mitte rechtwinkelig gebogen und durch eine Nafion 211 Membran getrennt. Anschließend wurden die MQ und die MHQ Lösungen in die jew. Seite eingefüllt. Die Messungen erfolgten im Potentiostatischen Modus ($\pm$0,75 V vs. Gegenelektrode, 1 h/Ladung/Entladung), als Elektroden wurden mittels Stahl kontaktierte Kohlenstoffpapierelektroden herangezogen. Der starke Abfall während den ersten Zyklen ist durch unerwünschte Nebenreaktionen und Blockung der Membran aufgrund der langen Laufzeit (ca. 10-20 h/Zyklus) erklärbar. Bemerkenswerter Weise ist nach 20 bzw. 40 Zyklen eine Steigerung der Kapazität erkennbar, solange die Zelle künstlich beleuchtet wird. Aufgrund von Diffusionslimitierung ist die Kapazität dieser Zelle unter der theoretischen Kapazität von 382 mAh/g.

***Beispiel 4:** Photochemie von 2-Methoxybenzochinon*

Wie in der Literatur beschrieben (Ononye AI et al. J Phys Chem 90(1986) :6266-6270; Karsili TNV et al. PCCP 17 (2015) :32183-32193; Bearnais-Barbry S et al. Photochem. Photobiol. 74 (2001) :542-8) können Benzochinone durch Photonenabsorption in einen angeregten Singlettzustand übergehen, von dem aus die via *"inter-system crossing"* in einen Tripplettzustand fallen können. Dieser ist relativ reaktiv und kann z.B. über die Reaktion mit Wasser und Luftsauerstoff zur Bildung die korrespondierenden Hydrochinons führen. Unglücklicher Weise sind auch andere Nebenreaktionen wie Dimerisierungen möglich, die zu kapazitativen Verlusten führen können. Während sich in HCl beinahe quantitativ das in Beispiel 1 diskutierte MQMHQ Dimer (und eventuell ein daraus gebildetes Dibenzofuran) bildet sind in Phosphorsäure weniger Nebenreaktionen beobachtbar.

Bei Zellversuchen in einer U-Zelle aus Glas konnte eine Abhängigkeit der Kapazität von der Lichteinstrahlung beobachtet werden. Bei Nutzung von einer aktiven Substanz mit niedrigerem Redoxpotenial auf der Gegenseite würde die Umsetzung vom Benzo- zum Hydrochinon einer Ladung der Halbzelle entsprechen. Daher wurden NMR-Experimente vor und nach Bestrahlung von MQ bzw. MHQ in $D_3PO_4$ mit einem Laser mit dem Emmissionsmaximum bei 355 nm durchgeführt. Während MHQ sich, wie erwartet, nicht verändert wird im Fall von MQ bereits nach 30 s Bestrahlung mit 3500 mW/cm$^2$ bei 365 nm eine Veränderung offensichtlich (Fig. 5a)). Dabei kann die Bildung von MHQ beobachtet werden. Zusätzlich traten v.a. dimerisierte Nebenprodukte auf, die nicht weiter charakterisiert wurden.

Mittels EPR Spektroskopie konnte der Mechanismus der Reaktion via Semichinon-radikal aufgeklärt werden. Die cw-EPR Spectren wurden mit einem Bruker X-band Spectrometer (EMX, 100 kHz field modulation)bei Raumtemperatur mit 0.02 mT field modulation amplitude aufgenommen. MQ Lösungen mit Konzentrationen zwischen 5 und 7 mM wurden in 4:1 Mischungen (V:V) von Acetonitril und wässrigen Phosphatpuffern bei pH = 3, pH = 4 and pH = 7.4, gelöst. Die gezeigten Spektren entsprechen den Gleichgewichtsbedingungen der Proben, die zirkuliert und kontinuierlich bestrahlt wurden (Hg-Xe UV Lampe, Hamamatsu Lightningcure LC4, 3500 mW/cm$^2$, Amax = 365 nm). Die simulierten Daten wurden mittels Winsim2002 software errechnet (siehe Fig. 6A und 6B).

***Beispiel 5:** Redox-Flow Batterie*

Die RFB Versuche wurden in einer LAB-1$\times$1 cell von C-Tech Innovation Ltd. (Chester, UK) durchgeführt (siehe Fig. 7). In die Reaktionszelle 4 wurden Kohlenstofffließelektroden 5a und 5b eingebracht (AvCarb 200). Als Membran 7 wurde eine Nafion 211 Proton exchange membrane verwendet. Für symmetrische Versuche wurden MHQ und MQ in 0,5M $H_3PO_4$ verwendet. Asymmetrische Versuche wurden mit MHQ und p-Benzochinon in 0,5 M $H_3PO_4$ durchgeführt. Die Lösungen wurden in Polypropylen-Tanks 2a und 2b gelagert und mittels Pumpen 3a und 3b mit 140 ml/s Fluss durch die Reaktionszelle 4 gepumpt. Der Fluss wurde mittels Arduino Uno ATmega328 Microcontrollern, Recom LED Treibern und RS pro flow Sensoren (0,05 - 10 l/min) mit WPDC-0.45L-3.1M-12 Kreiselpumpen eingestellt. Die Zyklisierung erfolgte im potentiostatischen ($\pm$0,75 V für jew. 1h/Zyclus, Fig. 8a) oder im galvanostatischn Modus (1C-5C Geschwindigkeit, Fig. 8b). Die Coulombals auch die Peakstromeffizienten bleiben über 250 Zyklen fast konstant bei 99% bzw. 92% (Fig. 9).

## Patentansprüche

1. Wässrige Zusammensetzung umfassend

a) mindestens eine Verbindung mit der allgemeinen Formel

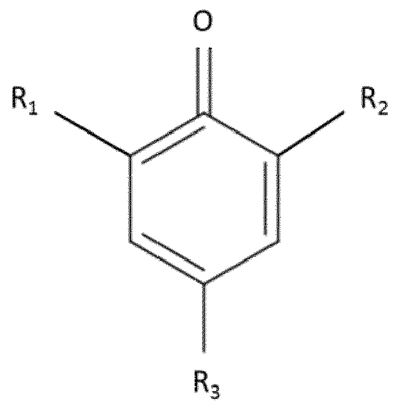

(I)

und mindestens eine Verbindung mit der allgemeinen Formel

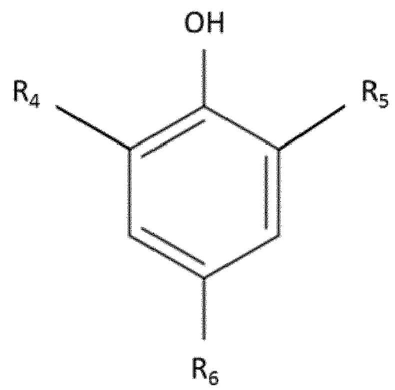

(II),

wobei $R_1$, $R_2$ und $R_3$ unabhängig voneinander O, H oder $OR_7$ und $R_4$, $R_5$ und $R_6$ unabhängig voneinander OH, H oder $OR_7$ sind, wobei $R_1$ O und $R_4$ OH, $R_2$ O und $R_5$ OH oder $R_3$ O und $R_6$ OH ist, wobei $R_7$ ein Alkyl- oder Allylrest ist, und

b) Phosphationen und/oder Hydrogenphosphationen und/oder deren Kondensate.

**2.** Zusammensetzung gemäß Anspruch 1, **dadurch gekennzeichnet, dass** die Verbindung der Formel (I) 2-Methoxybenzochinon (MQ) oder Parabenzochinon (pQ) und die Verbindung der Formel (II) 2-Methoxyhydrochinon (MHQ) oder Parahydrochinon (pHQ) ist.

**3.** Zusammensetzung gemäß Anspruch 1 oder 2, **dadurch gekennzeichnet, dass** die Kondensate der Phosphationen und Hydrogenphosphationen Hydrogendiphosphationen, Hydrogentriphosphationen, Hydrogenoligophosphationen oder Hydrogenpolyphosphationen sind.

**4.** Zusammensetzung gemäß einem der Ansprüche 1 bis 3, **dadurch gekennzeichnet, dass** die Zusammensetzung 0,0001 bis 1,2 mol/L, vorzugsweise 0,001 bis 1 mol/L, noch mehr bevorzugt 0,05 bis 0,75 mol/L, noch mehr bevorzugt 0,1 bis 0,75 mol/L, noch mehr bevorzugt 0,2 bis 0,7 mol/L, einer Verbindung der Formel (I) und/oder (II) umfasst.

**5.** Zusammensetzung gemäß einem der Ansprüche 1 bis 4, **dadurch gekennzeichnet, dass** die Zusammensetzung in Summe 0,01 bis 0,75 mol/L, vorzugsweise 0,05 bis 0,7 mol/L, noch mehr bevorzugt 0,1 bis 0,6 mol/L, noch mehr bevorzugt 0,15 bis 0,5 mol/L, Phosphationen und/oder Hydrogenphosphationen und/oder deren Kondensate umfasst.

**6.** Zusammensetzung gemäß einem der Ansprüche 1 bis 5, **dadurch gekennzeichnet, dass** die Zusammensetzung zusätzlich mindestens eine Säure mit einem pKs Wert von -7,5 bis 8, vorzugsweise - 3,5 bis 8, noch mehr bevorzugt

1,5 bis 8, noch mehr bevorzugt von 2 bis 7,5, noch mehr bevorzugt von 4,2 bis 6,8, noch mehr bevorzugt von 4,5 bis 6, umfasst.

7. Zusammensetzung gemäß Anspruch 6, **dadurch gekennzeichnet, dass** die Säure eine organische oder anorganische Säure ist, wobei die anorganische Säurevorzugsweise ausgewählt ist aus der Gruppe bestehend aus Salzsäure, Schwefelsäure, Salpetersäure und Phosphorsäure, und die organische Säure vorzugsweise ausgewählt ist aus der Gruppe bestehend aus Ameisensäure, Zitronensäure, Essigsäure, Propionsäure, Buttersäure, Glyoxalsäure, Malonsäure, Bernsteinsäure, Glutarsäure, Adipinsäure, Zitronensäure, Trimesinsäure, Trimellitsäure und Halbester davon.

8. Zusammensetzung gemäß Anspruch 6 oder 7, **dadurch gekennzeichnet, dass** die Zusammensetzung 0,001 bis 5 mol/L, vorzugsweise 0,005 bis 3 mol/L, Säure umfasst.

9. Zusammensetzung gemäß einem der Ansprüche 1 bis 8, **dadurch gekennzeichnet, dass** die Zusammensetzung mindestens einen Elektrolyten ausgewählt aus der Gruppe bestehend aus der Gruppe der Alkali- und Erdalkalimetallchloride, vorzugsweise NaCl, KCl oder $CaCl_2$, aus der Gruppe der Alkali- und Erdalkalimetallsulfate, vorzugsweise $Na_2SO_4$ oder $K_2SO_4$, und der Gruppe der Alkali- und Erdalkalimetallsalze von Carbonsäuren, Dicarbonsäuren und Tricarbonsäuren, vorzugsweise Natriumacetat, Natriumcitrat, Calciumcitrat oder Natriumoxalat, umfasst.

10. Zusammensetzung gemäß einem der Ansprüche 1 bis 9, **dadurch gekennzeichnet, dass** die Zusammensetzung mindestens einen Elektrolyten in einem Konzentrationsbereich von 0,001 bis 0,1 mol/L umfasst.

11. Redox-Flow Zelle umfassend eine wässrige Zusammensetzung gemäß einem der Ansprüche 1 bis 10.

12. Verfahren zur Reduktion einer Verbindung mit der allgemeinen Formel

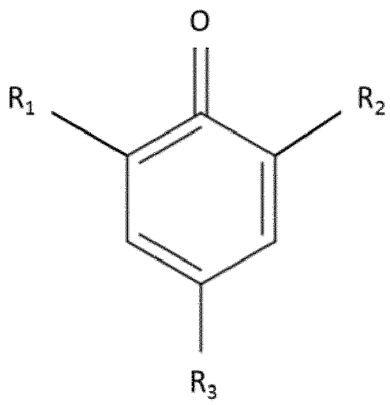

(I)

zu einer Verbindung mit der allgemeinen Formel

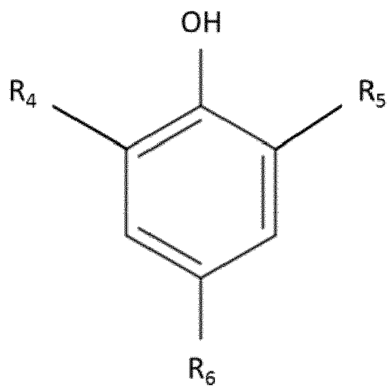

(II),

wobei $R_1$, $R_2$ und $R_3$ unabhängig voneinander O, H oder $OR_7$ und $R_4$, $R_5$ und $R_6$ unabhängig voneinander OH, H oder $OR_7$ sind, wobei $R_1$ O und $R_4$ OH, $R_2$ O und $R_5$ OH oder $R_3$ O und $R_6$ OH ist, wobei $R_7$ ein Alkyl- oder Allylrest ist, umfassend den Schritt des Inkontaktbringens einer wässrigen Zusammensetzung gemäß einem der Ansprüche 1 bis 12 mit Licht.

**13.** Verfahren gemäß Anspruch 12, **dadurch gekennzeichnet, dass** das Licht eine Wellenlänge von 190 bis 800 nm aufweist.

**14.** Verfahren gemäß Anspruch 12 oder 13, **dadurch gekennzeichnet, dass** die Reduktion in einem Temperaturbereich von 5 bis 65°C, vorzugsweise von 15 bis 55°C, noch mehr bevorzugt von 25 bis 35°C durchgeführt wird.

**15.** Verfahren gemäß einem der Ansprüche 12 bis 14, **dadurch gekennzeichnet, dass** die Reduktion in einer Redox-Flow Zelle durchgeführt wird.

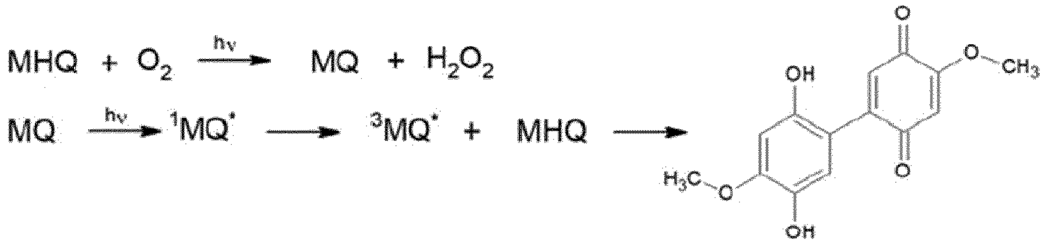
MHQ + $O_2$ hν → MQ + $H_2O_2$
MQ hν → $^1MQ^*$ → $^3MQ^*$ + MHQ →

Fig. 1

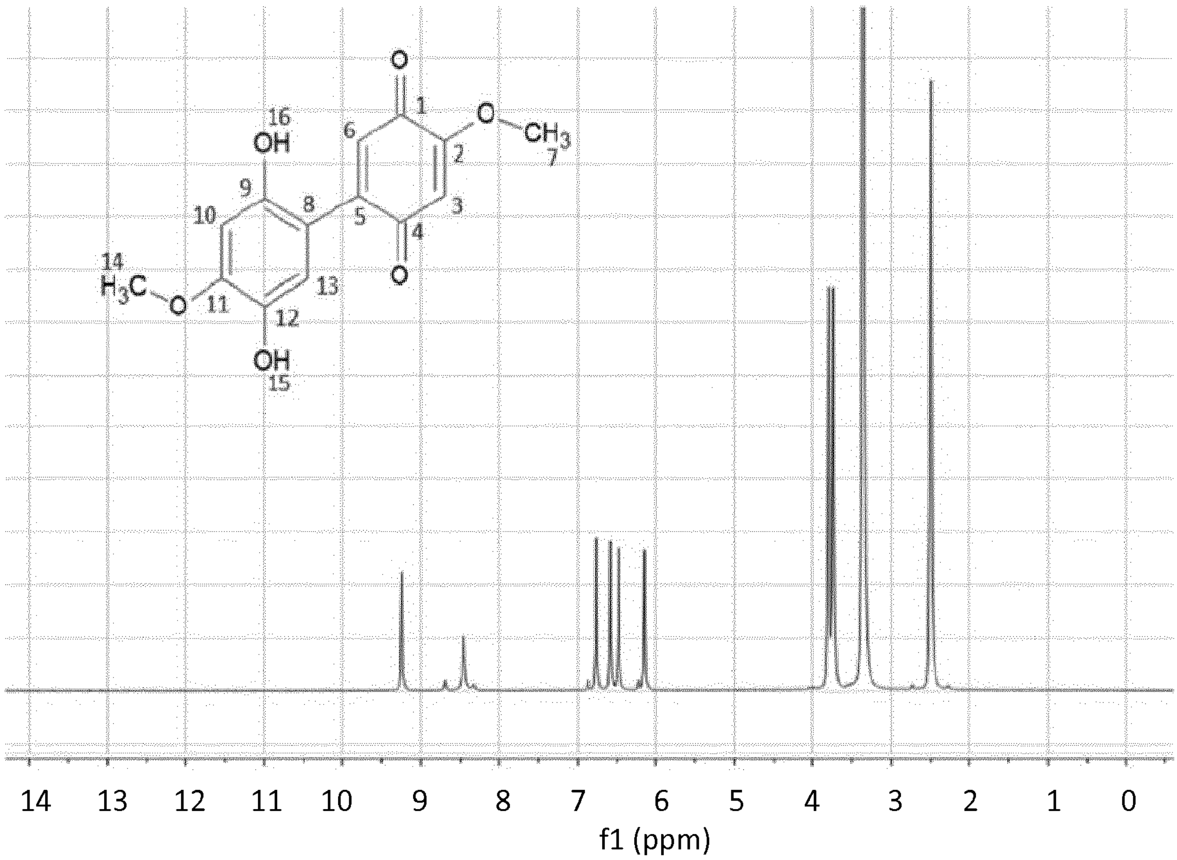
14 13 12 11 10 9 8 7 6 5 4 3 2 1 0
f1 (ppm)

Fig. 2

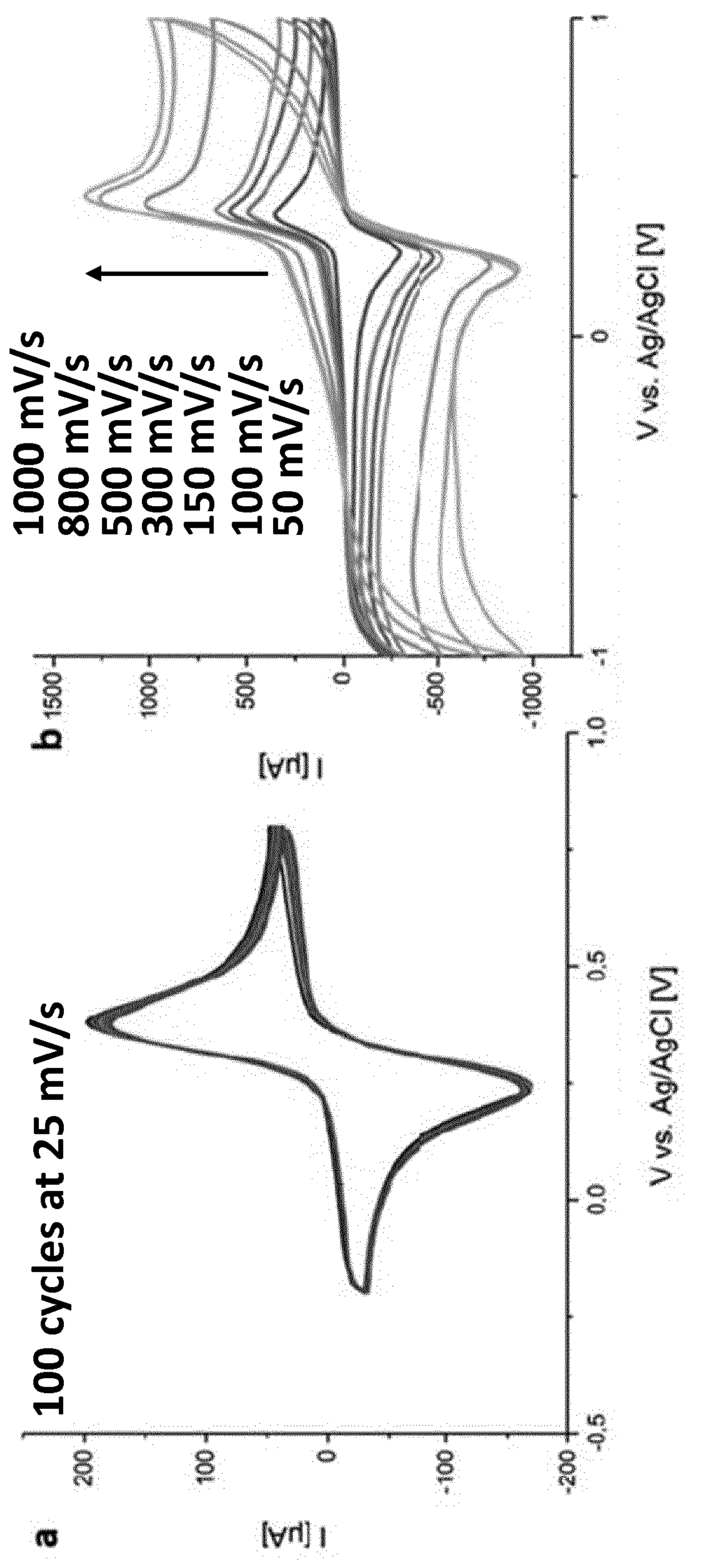
a
b
100 cycles at 25 mV/s
1000 mV/s
800 mV/s
500 mV/s
300 mV/s
150 mV/s
100 mV/s
50 mV/s
I [µA]
V vs. Ag/AgCl [V]
200
100
0
-100
-200
-0.5
0.0
0.5
1.0
1500
1000
500
-500
-1000
-1
1

Fig. 3

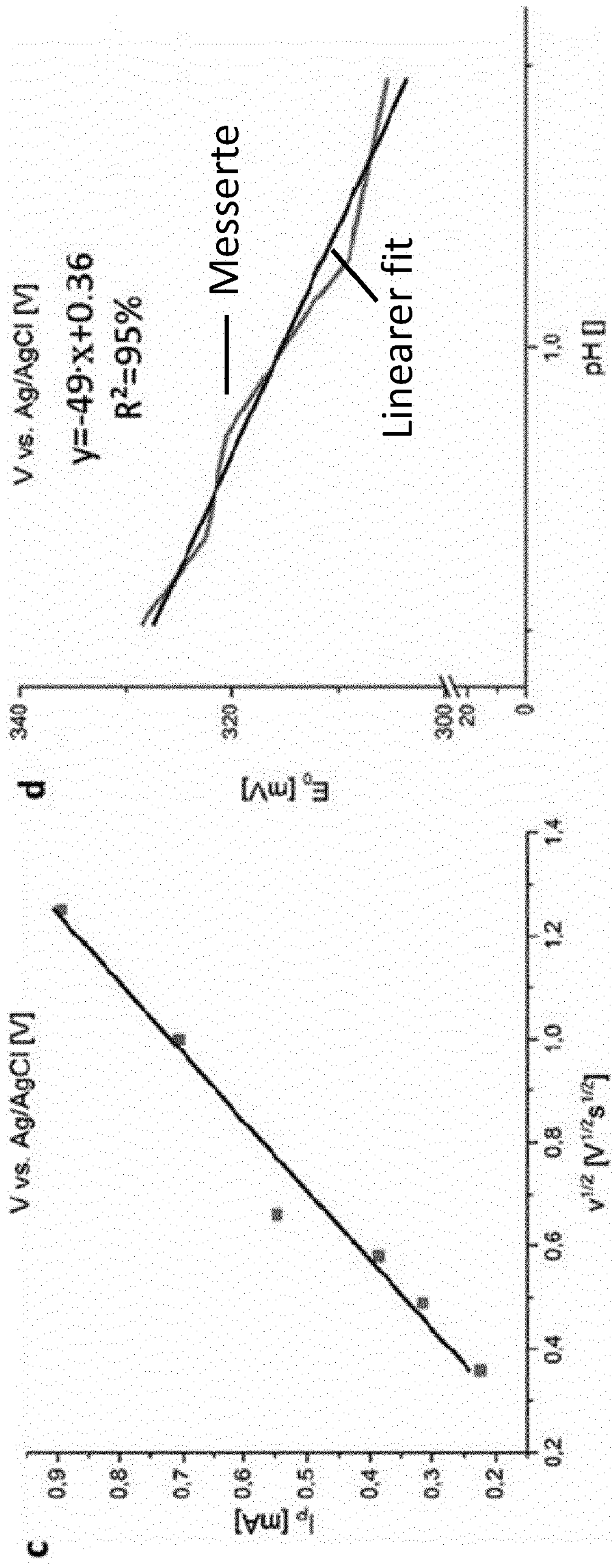


Fig. 3 (Fortsetzung)

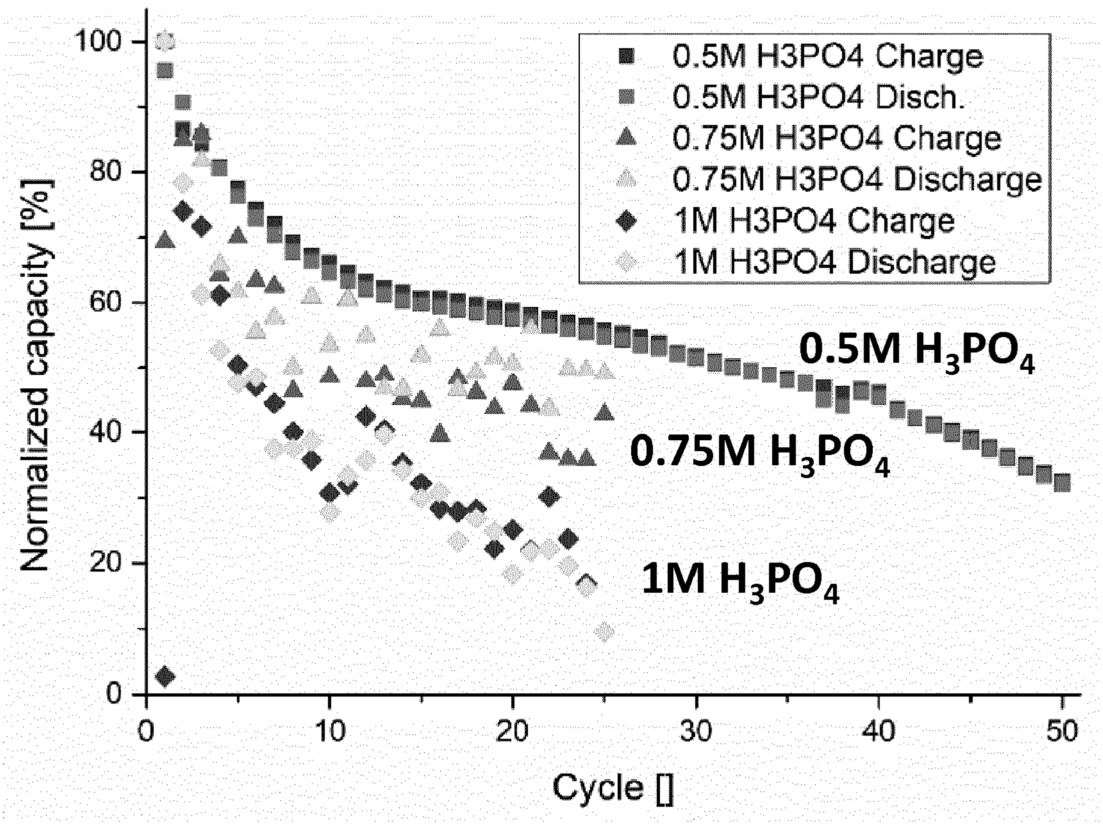
0.5M H3PO4 Charge
0.5M H3PO4 Disch.
0.75M H3PO4 Charge
0.75M H3PO4 Discharge
1M H3PO4 Charge
1M H3PO4 Discharge
0.5M $H_3PO_4$
0.75M $H_3PO_4$
1M $H_3PO_4$
Normalized capacity [%]
Cycle []

Fig. 4

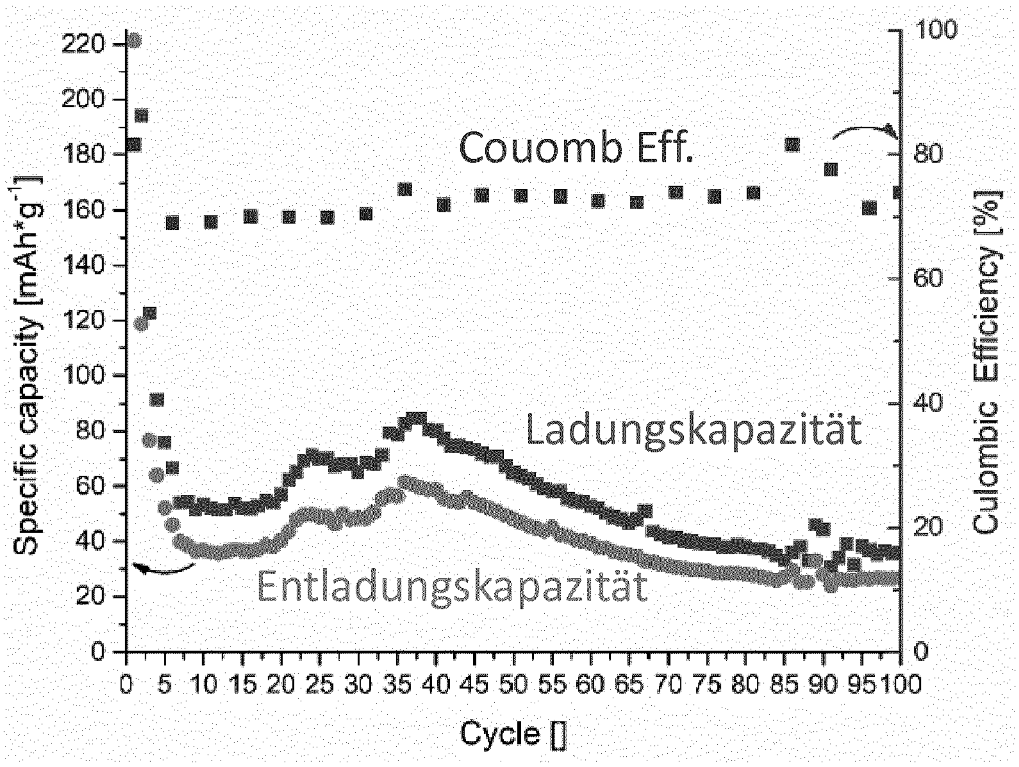
Couomb Eff.
Ladungskapazität
Entladungskapazität
Specific capacity [mAh*g$^{-1}$]
Culombic Efficiency [%]
Cycle []

Fig. 5

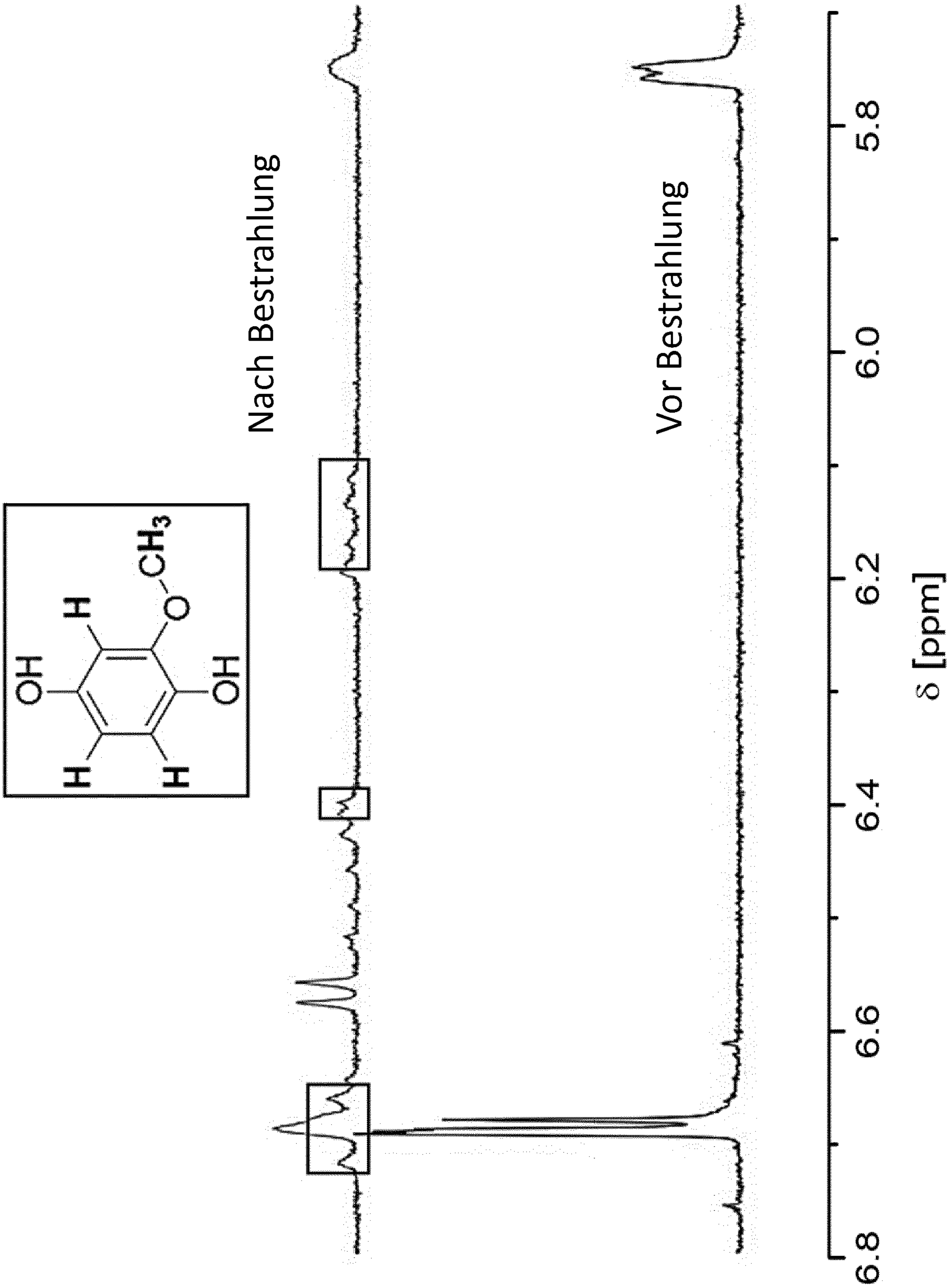


Fig. 6A

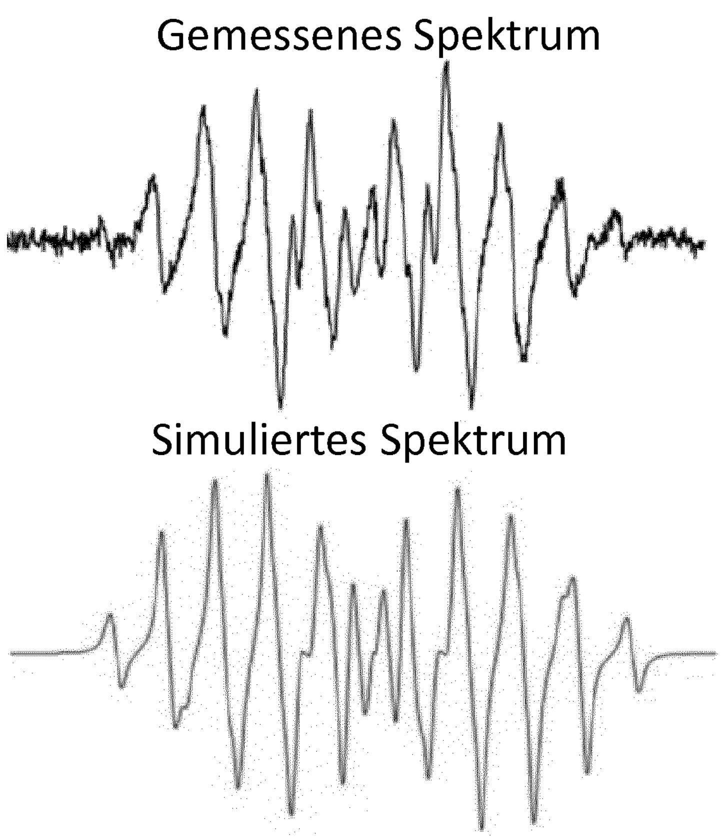
Gemessenes Spektrum
Simuliertes Spektrum

Fig. 6B

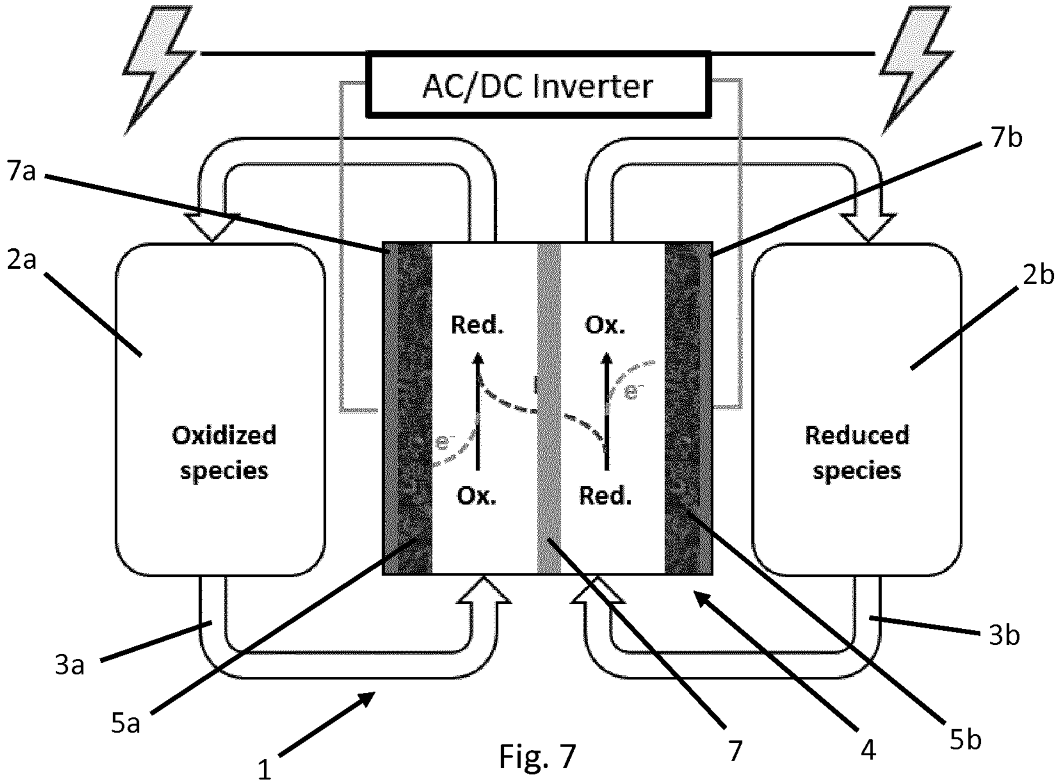
AC/DC Inverter
7a
7b
2a
2b
Red.
Ox.
e-
Oxidized species
Reduced species
Ox.
Red.
3a
3b
5a
5b
1
7
4

Fig. 7

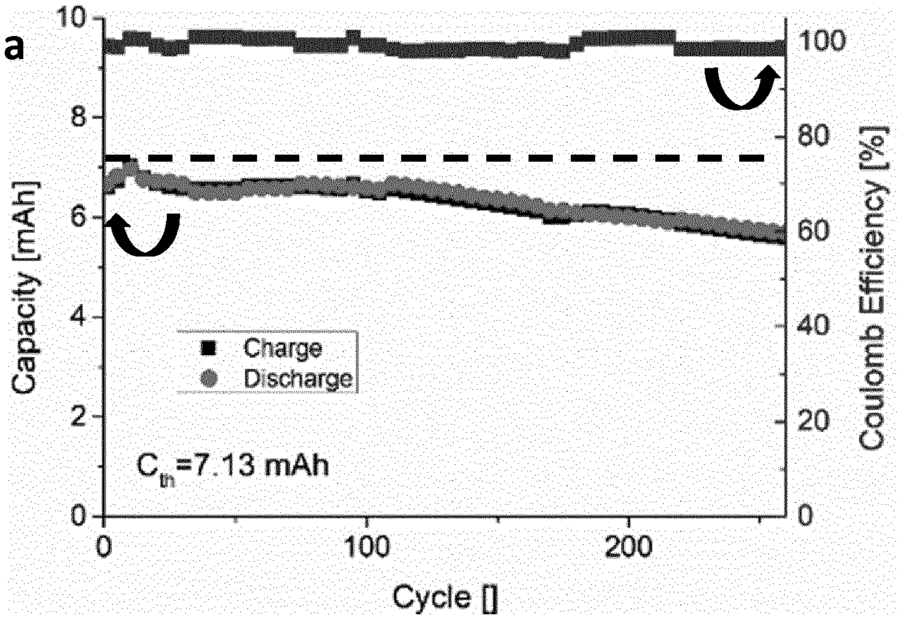
a
Capacity [mAh]
Coulomb Efficiency [%]
Charge
Discharge
$C_{th}$=7.13 mAh
Cycle []

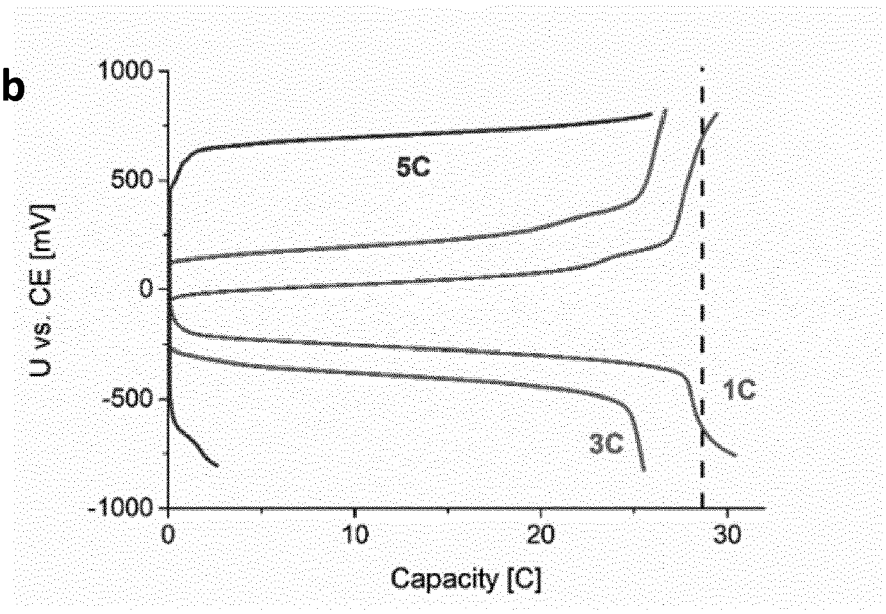
b
5C
3C
1C
U vs. CE [mV]
Capacity [C]

Fig. 8

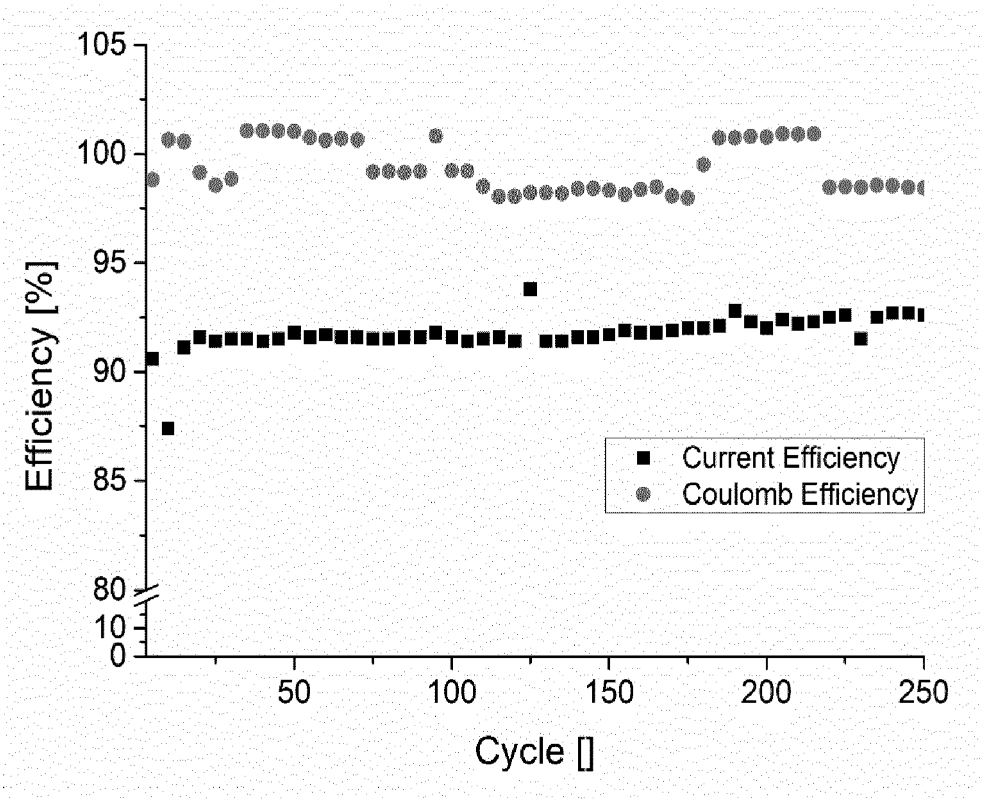
105
100
95
90
85
80
10
0
Efficiency [%]
50
100
150
200
250
Cycle []
Current Efficiency
Coulomb Efficiency

Fig. 9

Europäisches Patentamt
European Patent Office
Office européen des brevets

# EUROPÄISCHER RECHERCHENBERICHT

Nummer der Anmeldung
EP 19 21 2222

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 108 593 737 A (UNIV SCIENCE & TECHNOLOGY LIAONING) 28. September 2018 (2018-09-28) * Beispiel 8 * ----- | 1,4-8 | INV. H01M8/18 C07C46/06 |
| X | KR 2017 0037296 A (LG CHEMICAL LTD [KR]) 4. April 2017 (2017-04-04) * Ansprüche 9,15 * ----- | 1-15 | |
| X | WO 2015/023974 A1 (UNIV CALIFORNIA [US]; BIOSOLAR INC [US]) 19. Februar 2015 (2015-02-19) * Ansprüche 2,11 * ----- | 1,2,5 | |
| A | CN 102 035 007 A (63971 FORCES PLA) 27. April 2011 (2011-04-27) * Embodiment 3; Ansprüche 2,4 * ----- | 1-15 | |
| X | RO 120 939 B1 (MATACHE SAVEL [RO]) 29. September 2006 (2006-09-29) * Ansprüche 23,24; Beispiele 1-4 * ----- | 1,2,5,11 | RECHERCHIERTE SACHGEBIETE (IPC) |
| X | KRISTINA WEDEGE ET AL: "Organic Redox Species in Aqueous Flow Batteries: Redox Potentials, Chemical Stability and Solubility", SCIENTIFIC REPORTS, Bd. 6, Nr. 1, 1. Dezember 2016 (2016-12-01), XP055427452, DOI: 10.1038/srep39101 * Seite 2; Abbildung 3 * ----- | 1,2,5,11 | H01M C07C |
| A | WO 2019/158615 A1 (CMBLU PROJEKT AG [DE]) 22. August 2019 (2019-08-22) * Beispiel 4.2 * ----- | 1-15 | |
| | -/-- | | |

Der vorliegende Recherchenbericht wurde für alle Patentansprüche erstellt

1

| Recherchenort | Abschlußdatum der Recherche | Prüfer |
|---|---|---|
| München | 28. Mai 2020 | Steinreiber, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 19 21 2222

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A | ZHONG JIE ZHANG ET AL: "Illustrating the effect of electron withdrawing and electron donating groups adherent to p-hydroquinone on supercapacitor performance: The cases of sulfonic acid and methoxyl groups", ELECTROCHIMICA ACTA, Bd. 282, 15. Juni 2018 (2018-06-15), Seiten 563-574, XP055699523, AMSTERDAM, NL ISSN: 0013-4686, DOI: 10.1016/j.electacta.2018.06.061 * Abbildungen 1,8 * ----- | 1-15 | |
| | | | RECHERCHIERTE SACHGEBIETE (IPC) |

1 Der vorliegende Recherchenbericht wurde für alle Patentansprüche erstellt

| Recherchenort | Abschlußdatum der Recherche | Prüfer |
|---|---|---|
| München | 28. Mai 2020 | Steinreiber, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

Seite 2 von 2

## ANHANG ZUM EUROPÄISCHEN RECHERCHENBERICHT ÜBER DIE EUROPÄISCHE PATENTANMELDUNG NR.

EP 19 21 2222

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-05-2020

| Im Recherchenbericht angeführtes Patentdokument | Datum der Veröffentlichung | Mitglied(er) der Patentfamilie | Datum der Veröffentlichung |
|---|---|---|---|
| CN 108593737 A | 28-09-2018 | KEINE | |
| KR 20170037296 A | 04-04-2017 | KEINE | |
| WO 2015023974 A1 | 19-02-2015 | CA 2920365 A1 | 19-02-2015 |
| | | CN 105723482 A | 29-06-2016 |
| | | EP 3033758 A1 | 22-06-2016 |
| | | JP 2016532294 A | 13-10-2016 |
| | | KR 20160067837 A | 14-06-2016 |
| | | US 2016196929 A1 | 07-07-2016 |
| | | WO 2015023974 A1 | 19-02-2015 |
| CN 102035007 A | 27-04-2011 | KEINE | |
| RO 120939 B1 | 29-09-2006 | KEINE | |
| WO 2019158615 A1 | 22-08-2019 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **A.J. BARD.** Electrochemical methods: fundamentals and applications. Wiley, 1980
- **WEBER AZ et al.** *J. Appl. Electrochem.,* 2011, vol. 41, 1137
- **ONONYE AI et al.** *J Phys Chem,* 1986, vol. 90, 6266-6270
- **KARSILI TNV et al.** *PCCP,* 2015, vol. 17, 32183-32193
- **BEARNAIS-BARBRY S et al.** *Photochem. Photobiol.,* 2001, vol. 74, 542-8